# EUROPÄISCHE PATENTSCHRIFT

(11) **EP 1 406 599 B1**
(45) Veröffentlichungstag und Bekanntmachung des Hinweises auf die Patenterteilung: **15.04.2009**
(21) Anmeldenummer: 02784833.2
(22) Anmeldetag: 04.07.2002
(51) Int. Cl.: A61K 8/06, A61K 8/34, A61K 8/44, A61K 8/49, A61Q 19/00, A61Q 17/00

(54) **KREATIN ENTHALTENDE KOSMETISCHE UND DERMATOLOGISCHE ZUBEREITUNGEN ZUR BEHANDLUNG UND AKTIVEN PRÄVENTION TROCKENER HAUT UND ANDERER NEGATIVER VERÄNDERUNGEN DER PHYSIOLOGISCHEN HOMÖOSTASE DER GESUNDEN HAUT**
COSMETIC AND DERMATOLOGICAL PREPARATIONS CONTAINING CREATINE FOR TREATING AND ACTIVELY PREVENTING DRY SKIN AND OTHER NEGATIVE ALTERATIONS OF THE PHYSIOLOGICAL HOMEOSTASIS OF HEALTHY SKIN
PREPARATIONS COSMETIQUES ET DERMATOLOGIQUES A LA CREATINE UTILISEES DANS LE TRAITEMENT ET LA PREVENTION ACTIVE DE LA PEAU SECHE ET D'AUTRES MODIFICATIONS NEGATIVES DE L'HOMEOSTASE PHYSIOLOGIQUE DES PEAUX SAINES

(30) Priorität: 07.07.2001 DE 10133198
(43) Veröffentlichungstag der Anmeldung: 14.04.2004
(73) Patentinhaber: Beiersdorf AG, 20245 Hamburg (DE)
(72) Erfinder: SAUERMANN, Gerhard, 24649 Wiemersdorf (DE); SCHREINER, Volker, 20259 Hamburg (DE); DÖRING, Thomas, 41542 Dormagen (DE); SIEFKEN, Wilfried, 22529 Hamburg (DE); GATERMAN, Cornelia, 22179 Hamburg (DE); CARSTENSEN, Stefanie, 22303 Hamburg (DE); BIERGIESSER, Helga, 21465 Reinbek (DE); BLATT, Thomas, 22880 Wedel (DE)
(86) Internationale Anmeldenummer: PCT/EP2002/007405
(87) Internationale Veröffentlichungsnummer: WO 2003/005988

(56) Entgegenhaltungen:
- EP-A- 0 413 528
- WO-A-01/00203
- WO-A-02/02075
- WO-A-95/19769
- DE-A- 19 841 385
- US-A- 5 939 078

## Beschreibung

Die vorliegenden Erfindung betrifft kosmetische oder dermatologische zubereitungen enthalsend Kreatin und/oder bestimmte kreativ - Derivate zusammen mit einem oder mehreren Elektrolyten und mit einer oder mehreren Verbindungen aus Stoffgruppe kurzkettiger Polyole bzw. Urea allein oder in Kombination zur Behandlung und aktiven Prävention trockener Haut und zur Stärkung der Barrierefunktion der Haut und anderer negativer Veränderungen der physiologischen Homöostase der gesunden Haut.

Die Haut ist das größte Organ des Menschen. Unter ihren vielen Funktionen (beispielsweise zur Wärmeregulation und als Sinnesorgan) ist die Barrierefunktion, diejenige die das Austrocknen der Haut (und damit letztlich des gesamten Organismus) verhindert, die wohl wichtigste. Gleichzeitig wirkt die Haut als Schutzeinrichtung gegen das Eindringen und die Aufnahme von außen kommender Stoffe. Bewirkt wird diese Barrierefunktion durch die Epidermis, welche als äußerste Schicht die eigentliche Schutzhülle gegenüber der Umwelt bildet. Mit etwa einem Zehntel der Gesamtdicke ist sie gleichzeitig die dünnste Schicht der Haut.

Die Epidermis ist ein stratifiziertes Gewebe, in dem die äußere Schicht, die Hornschicht (*stratum corneum*), den für die Barrierefunktion bedeutenden Teil darstellt. Sie wird im Kontakt mit der Umwelt abgenutzt und befindet deshalb sich in einem ständigen Erneuerungsprozess, wobei nach außen kontinuierlich feine Schuppen abgegeben und von innen verhorntes Zell- und Lipidmaterial nachproduziert wird.

Das heute in der Fachwelt anerkannte Hautmodell von Elias (P. M. Elias, Structure and Function of the Stratum Corneum Permeability Barrier, Drug Dev. Res. 13, 1988, 97-105) beschreibt die Hornschicht als Zwei-Komponenten-System, ähnlich einer Ziegelsteinmauer (Ziegelstein-Mörtel-Modell). In diesem Modell entsprechen die Hornzellen (Komeozyten) den Ziegelsteinen, die komplex zusammengesetzte Lipidmembran in den Interzellularräumen entspricht dem Mörtel. Dieses System stellt im wesentlichen eine physikalische Barriere gegen hydrophile Substanzen dar, kann aber aufgrund seiner engen und mehrschichtigen Struktur gleichermaßen auch von lipophilen Substanzen nur schwer passiert werden. Die besondere Struktur der Hornschicht schützt einerseits die Haut und stabilisiert andererseits ihre eigene Flexibilität durch Bindung einer definierten Wassermenge.

Auch mechanische Belastungen, wie beispielsweise Druck-, Stoß- oder Scherkräfte, können in erstaunlichem Maße durch die Hornschicht allein oder im Verbund mit den tieferen Hautschichten abgefangen werden. Größere Druck-, Dreh- oder Scherkräfte werden über die Verzahnung der Epidermis mit dem Corium an tiefere Hautschichten weitergegeben.

Die Regulation des Wasser- und Feuchtigkeitsgehaltes ist eine der wichtigsten Funktionen der epidermalen Lipidmembran. Allerdings hat sie nicht nur eine Barrierewirkung gegen externe chemische und physikalische Einflüsse, sondern trägt auch zum Zusammenhalt der Hornschicht bei.

Die Lipide der Hornschicht bestehen im wesentlichen aus Ceramiden, freien Fettsäuren, Cholesterin sowie Cholesterinsulfat und sind über die gesamte Hornschicht verteilt. Die Zusammensetzung dieser Lipide ist für die intakte Funktion der epidermalen Barriere und damit für die Wasserundurchlässigkeit der Haut von entscheidender Bedeutung.

Bereits bei einer Reinigung der Haut mit Hilfe eines einfachen Wasserbads - ohne Zusatz von Tensiden - kommt es zunächst zu einer Quellung der Hornschicht der Haut. Der Grad dieser Quellung hängt u. a. von der Dauer des Bads und dessen Temperatur ab. Gleichzeitig werden wasserlösliche Stoffe ab- bzw. ausgewaschen, wie z. B. wasserlösliche Schmutzbestandteile, aber auch hauteigene Stoffe, die für das Wasserbindungsvermögen der Hornschicht verantwortlich sind. Durch hauteigene oberflächenaktive Stoffe werden außerdem auch Hautfette in gewissem Ausmaß gelöst und ausgewaschen. Dies bedingt nach anfänglicher Quellung eine nachfolgende Austrocknung der Haut, die durch waschaktive Zusätze noch deutlich verstärkt werden kann.

Bei gesunder Haut sind diese Vorgänge im allgemeinen belanglos, da die Schutzmechanismen der Haut solche leichten Störungen der oberen Hautschichten ohne weiteres kompensieren können. Aber bereits im Fall nichtpathologischer Abweichungen vom Normalstatus, z. B. durch umweltbedingte Abnutzungsschäden bzw. Irritationen, Lichtschäden, Altershaut usw., ist der Schutzmechanismus an der Hautoberfläche gestört.

Bei alter Haut beispielsweise erfolgt die regenerative Erneuerung verlangsamt, wobei insbesondere das Wasserbindungsvermögen der Hornschicht nachläßt. Sie wird deshalb inflexibel, trocken und rissig ("physiologisch" trockene Haut). Ein Barriereschaden ist die Folge. Die Haut wird anfällig für negative Umwelteinflüsse wie die Invasion von Mikroorganismen, Toxinen und Allergenen. Als Folge kann es sogar zu toxischen oder allergischen Hautreaktionen kommen.

Bei pathologisch trockener und empfindlicher Haut liegt ein Barriereschaden a priori vor. Epidermale Interzellularlipide werden fehlerhaft oder in ungenügender Menge bzw. Zusammensetzung gebildet. Die Konsequenz ist eine erhöhte Durchlässigkeit der Hornschicht und ein unzureichender Schutz der Haut vor Verlust an hygroskopischen Substanzen und Wasser.

Die Barrierewirkung der Haut kann über die Bestimmung des transepidermalen Wasserverlustes (TEWL - transepidermal water loss) quantifiziert werden. Dabei handelt es sich um die Abdunstung von Wasser aus dem Körperinneren ohne Einbeziehung des Wasserverlustes beim Schwitzen. Die Bestimmung des TEWL-Wertes hat sich als außerordentlich informativ erwiesen und kann zur Diagnose rissiger oder schrundiger Haut, zur Bestimmung der Verträglichkeit chemisch verschiedenartig aufgebauter Tenside und dergleichen mehr herangezogen werden.

Für die Schönheit und Gepflegtheit der Haut ist der Wasseranteil in der obersten Hautschicht von größter Bedeutung. Man kann ihn in einem begrenzten Umfang durch Einbringen von Feuchtigkeitsregulatoren günstig beeinflussen.

Anionische Tenside, welche im allgemeinen Bestandteile von Reinigungszubereitungen sind, können den pH-Wert in der Hornschicht langanhaltend erhöhen, was regenerative Prozesse, die der Wiederherstellung und Erneuerung der Barrierefunktion der Haut dienen, stark behindert. In diesem Fall stellt sich in der Hornschicht zwischen Regeneration und dem Verlust essentieller Substanzen durch regelmäßige Extraktion ein neuer, häufig sehr ungünstiger Gleichgewichtszustand ein, der das äußere Erscheinungsbild der Haut und die physiologische Funktionweise der Hornschicht entscheidend beeinträchtigt.

Unter Hautpflege im Sinne der vorliegenden Erfindung ist in erster Linie zu verstehen, daß die natürliche Funktion der Haut als Barriere gegen Umwelteinflüsse (z. B. Schmutz, Chemikalien, Mikroorganismen) und gegen den Verlust von körpereigenen Stoffen (z.B. Wasser, Lipide, Elektrolyte) gestärkt oder wiederhergestellt wird.

Produkte zur Pflege, Behandlung und Reinigung trockener und strapazierter Haut sind an sich bekannt. Allerdings ist ihr Beitrag zur Regeneration einer physiologisch intakten, hydratisierten und glatten Hornschicht umfangmäßig und zeitlich begrenzt.

Die Wirkung von Salben und Cremes auf die Barrierefunktion und die Hydratation der Hornschicht beruht im wesentlichen auf der Abdeckung (Okklusion) der behandelten Hautbezirke. Die Salbe oder Creme stellt sozusagen eine (zweite) künstliche Barriere dar, die den Wasserverlust der Haut verhindern soll. Entsprechend leicht kann diese physikalische Barriere - beispielsweise mit Reinigungsmitteln - wieder entfernt werden, wodurch der ursprüngliche, beeinträchtigte Zustand wieder erreicht wird. Darüber hinaus kann die Hautpflegewirkung bei regelmäßiger Behandlung nachlassen. Nach dem Absetzen der Produktanwendung kehrt die Haut sehr schnell wieder in den Zustand vor Behandlungsbeginn zurück. Bei bestimmten Produkten verschlechtert sich der Zustand der Haut unter Umständen sogar vorübergehend. Eine nachhaltige Produktwirkung wird in der Regel also nicht oder nur in einem eingeschränkten Maße erreicht.

Die Wirkung einiger pharmazeutischer Zubereitungen auf die Barrierefunktion der Haut besteht sogar in einer selektiven Barriereschädigung, die ermöglichen soll, daß Wirkstoffe in bzw. durch die Haut in den Körper eindringen können. Ein gestörtes Erscheinungsbild der Haut wird dabei als Nebenwirkung teilweise billigend in Kauf genommen.

Die Wirkung von pflegenden Reinigungsprodukten besteht im wesentlichen in einer effizienten Rückfettung mit Sebumlipid-ähnlichen Substanzen. Durch die gleichzeitige Verminderung des Tensidgehalts solcher Zubereitungen lässt sich der Schaden an der Hornschichtbarriere weiter begrenzen.

Dem Stand der Technik mangelt es allerdings an Zubereitungen, welche die Barrierefunktion und die Hydratation der Hornschicht positiv beeinflussen und die physikalisch-chemischen Eigenschaften der Hornschicht und insbesondere der Lamellen aus Interzellularlipiden stärken bzw. sogar wiederherstellen.

Um die Haut bei ihrer natürlichen Regeneration zu unterstützen und ihre physiologische Funktion zu stärken, werden den topischen Präparaten in neuerer Zeit zunehmend Interzellularlipidmischungen, wie Ceramide oder Ceramidanaloga zugesetzt, die von der Haut zum Wiederaufbau der natürlichen Barriere verwendet werden sollen. Allerdings handelt es sich bei diesen Lipiden zumeist um sehr teure Rohstoffe. Zudem ist ihre Wirkung meist geringer als erhofft.

Aufgabe der vorliegenden Erfindung war es also, die Nachteile des Standes der Technik zu beseitigen. Insbesondere sollten hautpflegende Zubereitungen zur Verfügung gestellt werden, welche die Barriereeigenschaften der Haut erhalten oder wiederherstellen, zumal dann, wenn die natürliche Regeneration der Haut nicht ausreicht. Sie sollen ferner zur Behandlung und Prophylaxe von Folgeschäden der Hautaustrocknung, beispielsweise Fissuren oder inflammatorischen oder allergischen Prozessen oder auch der Neurodermitis, geeignet sein. Aufgabe der vorliegenden Erfindung war es auch, stabile hautpflegende kosmetische und/oder dermatologische Mittel zur Verfügung zu stellen, welche die Haut vor Umwelteinflüssen wie Sonne und Wind schützen. Insbesondere sollte die Wirkung der Zubereitungen physiologisch, schnell und nachhaltig sein.

Weiterhin sollen Störungen der Homöostase der Haut, insbesondere der gesunden Haut, behandelt und behoben werden oder prophylaktisch behandelt werden.

Erfindungsgemäß werden die gestellten Aufgaben gelöst.

Diese Aufgaben werden überraschend und für den Fachmann nicht vorhersehbar gelöst durch kosmetische oder dermatologische Zubereitungen gemäß Anspruch 1, mit einem Gehalt an a) Kreatin, Kreatinphosphat, Kreatinsulfat, Kreatinacetat oder Kreatinascorbat b) 0,05 - 30 Gew.-% an einem oder mehreren Elektrolyten aus der Gruppe NaCl, NaBr, Nal, Na₂B₄O₇, Na₂SiO₃, Na₂CO₃, NaHCO₃, Na₃PO₄, Na₂HPO₄, NaH₂PO₄, KCl, Kl, LiCl, NH₄Cl, ZnCl₂, Al₂SO₄, MgSO₄, Natriumliponat, Natriumcitrat, Ammoniumlactat, Natriumlactat, Natriumbicarbonat, Natriumcitrat, Natriumpropionat, bezogen auf das Gesamtgewicht der Zubereitungen c) einer oder mehreren Substanzen gewählt aus der Gruppe Harnstoff und 0,05 - 30 Gew.- % an Polyolen, gewählt aus der Gruppe Glycerin, Butylenglykole, Propylenglykole, Ethylenglykol, Pentandiole, Hexandiole, insbesondere jeweils die vicinalen Hydroxyverbindungen, Diethylenglykol, Triethylenglykol, Dipropylenglykol, Tripropylenglykol, Dibutylenglykol oder Tributylenglykol, bezogen auf das Gesamtgewicht der Zubereitungen.

Die Dokumente DE-A-198 41 385, US-A-5 939 078, WO-A-01/00203, EP-A- 0 413 528, WO-A-95/19769 und WO-A-02/02075 beschreiben zwar Zubereitungen, die einzelne der erfindungsgemäßen Wirkstoffe enthalten, geben aber keinen Hinweis auf die erfindungsgemäßen Wirkstoffkombinationen.

Die Zubereitungen sind geeignet zur Behandlung und aktiven Prävention trockener Haut und zur Stärkung der Barrierefunktion der Haut sowie zur Behandlung, Pflege und Prophylaxe von
sensibler Haut und/oder zur Behandlung und Prophylaxe der Symptome einer negativen Veränderung der physiologischen Homöostase der gesunden Haut, insbesondere defizitärer, sensitiver oder hypoaktiver Hautzustände oder defizitärer, sensitiver oder hypoaktiver Zustände von Hautanhangsgebilden,
entzündlicher Hautzustände sowie des atopischen Ekzems, der polymorphen Lichtdermatose, Psoriasis, Vitiligo,
empfindlicher, juckender oder gereizter Haut,
Veränderungen der normalen Lipidperoxidation,
einer Veränderung des Ceramid-, Lipid- und Energiestoffwechsels der gesunden Haut,
einer Veränderung des physiologischen transepidermalen Wasserverlustes,
einer Verminderung der Hauthydratation und Abnahme des Feuchtigkeitsgehaltes der Haut,
Veränderung des Natural Moisturizing Factor Gehaltes,
Verminderung der Zell-Zell-Kommunikation,
Mangelerscheinungen der intrazellulären DNS-Synthese,
DNS-Schädigungen und Verminderung von endogenen DNS-Reparaturmechanismen,
Aktivierung von Metalloproteinasen und/oder anderer Proteasen bzw. Inhibierung der entsprechenden endogenen DNS-Reparaturmechanismen,
Abweichungen von den normalen post-translationalen Modifikationen von Bindegewebsbestandteilen,
Veränderungen des normalen Hyaluronsäure- und Glucosaminoglycangehalts der gesunden Haut, der Schuppenbildung der Haare, der Schuppenbildung der Kopfhaut und der Hautalterung.

Der Aufbau des menschlichen Haares gleicht in Grundzügen dem der Hornschicht der menschlichen Haut. Zwischen den abgestorbenen Komeozyten befinden sich Lipide, wie z.B. Ceramide, die der Austrocknung und Strukturschwäche des Haares entgegenwirken. Daher kann durch die erfindungsgemäßen Wirkstoffe und deren Kombinationen auch die Haarstruktur verbessert werden. Darüberhinaus sind die erfindungsgemäßen Wirkstoffe und deren Kombinationen auch zur Behandlung einer schuppenden Kopfhaut geeignet.

Die Alterung der Haut, insbesondere wenn durch chronische Sonneneinstrahlung gefördert, stellt z.B. eine besonders dramatische Form der Störung der Hauthomöostase dar. Überraschenderweise verbessern die erfindungsgemäßen Wirkstoffe und deren Kombinationen ganz besonders homöostatische Abweichungen der Altershaut. Sie sind daher, wie auch die Zubereitungen, die sie enthalten, zur Behandlung und prophylaktischen Behandlung der Hautalterung sehr gut geeignet.

Die erfindungsgemäßen Verbindungen können gegebenenfalls als Säuren oder in Form ihrer Salze verwendet werden, z B. der wasserlöslichen Salze, z.B. der Natrium- oder Kaliumsalze .

Ein besonders bevorzugtes Derivat des Kreatins ist das Kreatinphosphat (Phosphokreatin).

Weitere bevorzugte Derivate sind Kreatinsulfat, Kreatinacetat oder Kreatinascorbat.

Wirkstoffe der Gruppe a) sind erfindungsgemäß vorteilhaft in kosmetischen oder dermatologischen Zubereitungen z. B. zu 0,001 Gew.% bis 30 Gew.-%, bevorzugt zu 0,05 Gew.-% bis 10 Gew.-%, insbesondere bevorzugt zu 0,1 - 5,0 Gew.-%, bezogen auf das Gesamtgewicht der Zubereitungen, enthalten.

Geeignete Elektrolyte werden ausgewählt aus der Gruppe NaCl, NaBr, NaI, Na₂B₄O₇, Na₂SiO₃, Na₂CO₃, NaHCO₃, Na₃PO₄, Na₂HPO₄, NaH₂PO₄, KCI, KI, LiCl, NH₄Cl, ZnCl₂, Al₂SO₄ und MgSO₄, Natriumliponat, Natriumcitrat, Ammoniumlactat, Natriumlactat, Natriumbicarbonat, Natriumcitrat Überrachenderweise Natriumpropionat stimuliert das genannte Wirksystem den hauteigenen Stoffwechsel von Lipiden und Proteinen, die zur Aufrechterhaltung der epidermalen Barriere für Wasser ständig neu gebildet werden müssen. Erfindungsgemäß wird insbesondere die trockene Haut durch die barrierestärkende Wirkung dieser Präparate behandelt und/oder gepflegt, während eine Austrocknung der normalen Haut aktiv verhindert wird.

Die kosmetischen oder dermatologischen Zubereitungen gemäß der Erfindung enthalten 0,05 - 30 Gew.-%, bevorzugt 1 - 5 Gew.-%, an einem oder mehreren Elektrolyten, bevorzugt Natriumchlorid, bezogen auf die Gesamtzusammensetzung der Zubereitungen. Die Substanzen der Gruppe c) werden gewählt aus der Gruppe Harnstoff und den Polyolen Glycerin, Butylenglykole, Propylenglykole, Ethylenglykol, Pentandiole, Hexandiole, insbesondere jeweils die vicinalen Hydroxyverbindungen, Diethylenglykol, Triethylenglykol, Dipropylenglykol, Tripropylenglykol, Dibutylenglykol und Tributylenglykol.

Polyole sind in kosmetischen oder dermatologischen Zubereitungen zu 0,05 Gew.-% bis 30 Gew.-%, bevorzugt zu 0,1 Gew.-% bis 20 Gew.-%, insbesondere bevorzugt zu 1 - 15 Gew.-%, bezogen auf das Gesamtgewicht der Zubereitungen, enthalten.

Harnstoff ist erfindungsgemäß vorteilhaft in kosmetischen oder dermatologischen Zubereitungen z.B. zu 0,05 Gew.-% bis 30 Gew.-%, bevorzugt zu 0,1 Gew.-% bis 20 Gew.-%, insbesondere bevorzugt zu 1 - 15 Gew.-%, bezogen auf das Gesamtgewicht der Zubereitungen, enthalten.

Bei der Kombination von Polyol und Harnstoff sind diese Stoffe erfindungsgemäß vorteilhaft in kosmetischen oder dermatologischen Zubereitungen z.B. zu 0,05 Gew.-% bis 30 Gew.-%, bevorzugt zu 0,1 Gew.-% bis 20 Gew.-%, insbesondere bevorzugt zu 1 - 15 Gew.-%, bezogen auf das Gesamtgewicht der Zubereitungen, enthalten.

Das Verhältnis des Gewichtes der Wirkstoffe der Gruppe b) (Elektrolyte) zum Gewicht der Wirkstoffe der Gruppe c) kann variieren. Beispielsweise kann das Gewichtsverhältnis b) / c) 10:1 bis 1:10 betragen, vorzugsweise 2:1 bis 1:2, insbesondere aber 1:1.

Bei der Kombination von Polyol und Harnstoff kann das Verhältnis des Gewichtes des Polyols zum Gewicht des Harnstoffs variieren. Beispielsweise kann das Gewichtsverhältnis Polyol/Harnstoff 1:10 bis 10:1 betragen, vorzugsweise 2:1 bis 1:2, insbesondere aber 1:1.

Überrachenderweise stimuliert das genannte Wirksystem den hauteigenen Stoffwechsel von Lipiden und Proteinen, die zur Aufrechterhaltung der epidermalen Barriere für Wasser ständig neu gebildet werden müssen. Erfindungsgemäß wird die trockene Haut durch die barrierestärkende Wirkung dieser Präparate behandelt und/oder gepflegt, während eine Austrocknung der normalen Haut aktiv verhindert wird.

Die Zubereitungen gemäß der Erfindung sind in jeglicher Hinsicht überaus befriedigende Präparate. Es war für den Fachmann nicht vorauszusehen, dass die Zubereitungen gemäß der Erfindung
- besser die Barriereeigenschaften der Haut erhalten oder wiederherstellen,
- die Ceramidbiosynthese der Haut verstärken,
- besser der Hautaustrocknung entgegenwirken,
- besser gegen die Hautalterung wirken und
- die Haut besser vor Umwelteinflüssen schützen
   als die Zubereitungen des Standes der Technik.

Die kosmetischen oder dermatologischen Zubereitungen gemäß der Erfindung können wie üblich zusammengesetzt sein und zur Behandlung, Pflege und Reinigung der Haut und/oder der Haare und als Schminkprodukt in der dekorativen Kosmetik dienen. Entsprechend können sie, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die Zubereitungen gemäß der Erfindung als Grundlage für pharmazeutische Formulierungen zu verwenden. Die erfindungsgemäßen Zubereitungen enthalten z.B. 0,001 bis 30 Gew.- %, bevorzugt 0,01 Gew.-% bis 10 Gew.-%, insbesondere aber 0,1 Gew.-% bis 5 Gew.- %, jeweils bezogen auf das Gesamtgewicht der Zubereitungen an den erfindungsgemäßen Wirkstoffen.

Besonders bevorzugt werden die erfindungsgemäß verwendeten Wirkstoffkombinationen in pH-gepufferten Zubereitungen eingesetzt, wobei ein pH-Bereich von 5-7, insbesondere etwa 5-6 ganz besonders bevorzugt wird.

Günstig sind auch solche kosmetischen und dermatologischen Zubereitungen, die in der Form eines Sonnenschutzmittels vorliegen. Vorzugsweise enthalten diese neben einem oder mehreren erfindungsgemäßen Wirkstoffen mindestens eine UV-A-Filtersubstanz und/oder mindestens eine UV-B-Filtersubstanz und/oder mindestens ein anorganisches Pigment.

Es ist aber auch vorteilhaft im Sinne der vorliegenden Erfindung, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z.B. in Tagescrèmes gewöhnlich UV-A- bzw. UV-B-Filtersubstanzen eingearbeitet.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, organische Lösemittel oder Silikonderivate.

Die jeweils einzusetzenden Mengen an kosmetischen, dermatologischen oder medizinischen Trägerstoffen und Parfüm können in Abhängigkeit von der Art des jeweiligen Produktes vom Fachmann durch einfaches Ausprobieren leicht ermittelt werden.

Zubereitungen zur Behandlung und Pflege der Haut werden besonders bevorzugt.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Erfindungsgemäße kosmetische und dermatologische Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, einen festen Stift, eine Salbe oder auch ein Aerosol darstellen. Es ist auch vorteilhaft, die erfindungsgemäßen Wirkstoffe in verkapselter Form darzureichen, z.B. in Kollagenmatrices und anderen üblichen Verkapselungsmaterialien, z.B. als Celluloseverkapselungen, in Gelatine, Wachsmatrices oder liposomal verkapselt.

Es ist auch möglich und vorteilhaft im Sinne der vorliegenden Erfindung, die erfindungsgemäßen Wirkstoffe in wäßrige Systeme bzw. Tensidzubereitungen zur Reinigung der Haut und der Haare einzufügen.

Insbesondere können die erfindungsgemäßen kosmetischen und dermatologischen Zubereitungen auch Antioxidantien enthalten. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Carnosin, D-Camosin, L-Carnosin und deren Derivate (z. B. Anserin), Carotinoide, Carotine (z. B. α-Carotin, β-Carotin, ψ-Lycopin) und deren Derivate, Chlorogensäure und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glykosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, Vitamin C und Derivate (z. B. Ascorbylpalmitat, Mg-Ascorbylphosphat, Ascorbylacetat), Tocopherole und Derivate (z. B. Vitamin-E-acetat), Vitamin A und Derivate (Vitamin-A-palmitat) sowie Koniferylbenzoat des Benzoeharzes, Rutinsäure und deren Derivate, α-Glykosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, ZnSO₄), Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den erfindungsgemäßen Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Vorteilhafte Emulgatoren sind beispielsweise Glycerylstearat im Gemisch mit Ceteareth-20; Sorbitanstearat; Sorbitanoleat; Ceteareth-25; Ceteareth-6 im Gemisch mit Stearylalcohol; Cetylstearylalcohol im Gemisch mit PEG-40-Ricinusöl und Natriumcetylstearylsulfat; Triceteareth-4 Phosphat; Glycerylstearat; Natriumcetylstearylsulfat; Lecithin; Trilaureth-4 Phosphat; Laureth-4 Phosphat; Stearinsäure; Propylen Glycolstearat SE; PEG-25-hydriertes Ricinusöl; PEG-54-hydriertes Ricinusöl; PEG-6 Caprylsäure/Caprinsäureglyceride; Sorbitanstearat; Glyceryloleat im Gemisch mit Propylenglycol; PEG-9-Stearat; Glyceryllanolat; Ceteth-2; Ceteth-20; Polysorbat 60; Lanolin; Glycerylstearat im Gemisch mit PEG-100 Stearat; Glycerylmyristat; mikrokristallines Wachs (Cera microcristallina) im Gemisch mit Paraffinöl (Paraffinum liquidum), Ozokerit, hydriertem Ricinusöl, Glyceryl Isostearat und Polyglyceryl-3-Oleat; Glyceryllaurat; PEG-40-Sorbitanperoleat; Laureth-4; Ceteareth-3; Wollwachssäuregemische; Isostearylglycerylether; Cetylstearylalkohol im Gemisch mit Natrium Cetylstearylsulfat; Wollwachsalkoholgemische; Laureth-23; Steareth-2; Glycerylstearat im Gemisch mit PEG-30 Stearat; PEG-40-Stearat; Glycol Distearat; PEG-22-Dodecyl Glycol Copolymer; Polyglyceryl-2-PEG-4 Stearat; Pentaerythrithylisostearat; Polyglyceryl-3 Diisostearat; Ceteareth-20; Sorbitan Oleat im Gemisch mit hydriertem Ricinusöl, Bienenwachs (Cera alba) und Stearinsäure; Natriumdihydroxycetylphosphat im Gemisch mit Isopropylhydroxycetylether; Methylglucosesesquistearat; Steareth-10; PEG-20-Stearat; Steareth-2 im Gemisch mit PEG-8 Distearat; Steareth-21; Steareth-20; Isosteareth-20; Methylglucosedioleat; PEG-7-hydriertes Ricinusöl; Sorbitanoleat im Gemisch mit PEG-2-hydriertem Ricinusöl, Ozokerit und hydriertem Ricinusöl; Sorbitanisostearat im Gemisch mit PEG-2-hydriertem Ricinusöl, Ozokerit und hydriertem Ricinusöl; PEG-45/ Dodecylglycol-Copolymer; Methoxy-PEG-22/Dodecylglycol-Copolymer; hydrierte Cocosfettsäureglyceride; Polyglyceryl-4-Isostearat; PEG-40-Sorbitanperoleat; PEG-40-Sorbitanperisostearat; PEG-20-Glycerylstearat; PEG-20-Glycerylstearat; PEG-8-Bienenwachs; Laurylmethiconcopolyol; Cetyldimethiconcopolyol; Polyglyceryl-2-laurat; Isostearyldiglycerylsuccinat; Stearamidopropyl-PG-dimoniumchloridphosphat; PEG-7-hydriertes Ricinusöl; Glycerylstearat, Ceteth-20; Triethylcitrat; PEG-20-Methylglucosesesquistearat; Ceteareth-12; Paraffinöl (Paraffinum liquidum); Glycerylstearatcitrat; Cetylphosphat; Sorbitansesquioleat; Acrylat/C₁₀₋₃₀-Alkylacrylat-Crosspolymer; Sorbitanisostearat; Methylglucosesesquistearat; Triceteareth-4-Phosphat; Trilaureth-4-Phosphat; Polyglycerylmethylglucosedistearat; Poloxamer 101; Kaliumcetylphosphat; Isosteareth-10; Polyglyceryl-2-sesquiisostearat; Ceteth-10; Polyglyceryl-2 Dipolyhydroxystearat; Oleth-20; Isoceteth-20; Glycerylisostearat; Polyglyceryl-3-Diisostearat; Glycerylstearat im Gemisch mit Ceteareth-20, Ceteareth-12, Cetylstearylalcohol und Cetylpalmitat; Cetylstearylalcohol im Gemisch mit PEG-20 Stearat; Glycerylstearat; PEG-30-Stearat.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, - monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Als Treibmittel für erfindungsgemäße, aus Aerosolbehältern versprühbare Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Enthalten die erfindungsgemäßen Zubereitungen UVB-Filtersubstanzen, können diese öllöslich oder wasserlöslich sein. Erfindungsgemäß vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester,
- 2,4,6-Trianilino-(p-carbo-2'-ethyl-1'-hexyloxy) -1,3,5-triazin.

Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze sowie das 1,4-di(2-oxo-10-Sulfo-3-bornylidenmethyl)-Benzol und dessen Salze (die entprehenden 10-Sulfatoverbindungen, beispielsweise das entsprechende Natrium-, Kalium- oder Triethanolammonium-Salz), auch als Benzol-1,4-di(2-oxo-3-bornylidenmethyl-10-Sulfonsäure bezeichnet

Die Liste der genannten UVB-Filter, die in Kombination mit den erfindungsgemäßen Wirkstoffkombinationen verwendet werden können, soll selbstverständlich nicht limitierend sein.

Es kann auch von Vorteil sein, die erfindungsgemäß verwendeten Wirkstoffkombinationen mit UVA-Filtem zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Kosmetische und dermatologische Zubereitungen mit einem wirksamen Gehalt an erfindungsgemäß verwendeten Wirkstoffkombinationen können auch anorganische Pigmente enthalten, die üblicherweise in der Kosmetik zum Schutze der Haut vor UV-Strahlen verwendet werden. Dabei handelt es sich um Oxide des Titans, Zinks, Zirkoniums, Siliciums, Mangans, Cers und Mischungen davon, sowie Abwandlungen, bei denen die Oxide die aktiven Agentien sind. Besonders bevorzugt handelt es sich um Pigmente auf der Basis von Titandioxid.

Bei kosmetischen und dermatologischen Zubereitungen zum Schutze der Haare vor UV-Strahlen gemäß der Erfindung handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen und dermatologischen enthalten Wirkstoffe und Hilfsstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Verdickungsmittel, Emulgatoren, Fette, Öle, Wachse, organische Lösungsmittel, Bakterizide, Parfüme, Farbstoffe oder Pigmente, deren Aufgabe es ist, die Haare oder die kosmetische oder dermatologische Zubereitung selbst zu färben.

Die erfindungsgemäßen Anionen werden bevorzugt gewählt aus der Gruppe der Chloride, der Sulfate und Hydrogensulfate, der Phosphate, Hydrogenphosphate und der linearen und cyclischen Oligophosphate sowie der Carbonate und Hydrogencarbonate.

Kosmetische Zubereitungen, die ein Hautreinigungsmittel oder Shampoonierungsmittel darstellen, enthalten vorzugsweise mindestens eine anionische, nicht-ionische oder amphotere oberflächenaktive Substanz, oder auch Gemische aus solchen Substanzen, die erfindungsgemäß verwendeten Wirkstoffkombinationen im wäßrigen Medium und Hilfsmittel, wie sie üblicherweise dafür verwendet werden. Die oberflächenaktive Substanz bzw. die Gemische aus diesen Substanzen können in einer Konzentration zwischen 1 Gew.-% und 50 Gew.-% in dem Shampoonierungsmittel vorliegen.

Liegen die kosmetischen oder dermatologischen Zubereitungen in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um wäßrige oder wäßrig-alkoholische Lösungen, die gegebenenfalls oberflächenaktive Substanzen enthalten, deren Konzentration zwischen 0,1 und 10 Gew.-%, vorzugsweise zwischen 0,2 und 5 Gew.-%, liegen kann.

Diese kosmetischen oder dermatologischen Zubereitungen können auch Aerosole mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nicht-ionisches oder amphoteres Polymer oder auch Gemische derselben, sowie erfindungsgemäß verwendete Wirkstoffkombinationen in wirksamer Konzentration. Die Menge der verwendeten Polymeren liegt z.B. zwischen 0,1 und 10 Gew.-%, bevorzugt zwischen 0,1 und 3 Gew.-%.

Kosmetische Zubereitungen zur Behandlung und Pflege der Haare, die die erfindungsgemäß verwendeten Wirkstoffkombinationen enthalten, können als Emulsionen vorliegen, die vom nicht-ionischen oder anionischen Typ sind. Nicht-ionische Emulsionen enthalten neben Wasser Öle oder Fettalkohole, die beispielsweise auch polyethoxyliert oder polypropoxyliert sein können, oder auch Gemische aus den beiden organischen Komponenten. Diese Emulsionen enthalten gegebenenfalls kationische oberflächenaktive Substanzen.

Erfindungsgemäß können kosmetische Zubereitungen zur Behandlung und Pflege der Haare als Gele vorliegen, die neben einem wirksamen Gehalt an erfindungsgemäßen Wirkstoffen und gegebenenfalls dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Aluminiumsilikate wie beispielsweise Bentonite, oder ein Gemisch aus Polyethylenglykol und Polyethylenglycolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Vorzugsweise beträgt die Menge an erfindungsgemäßen Wirkstoffen in einem für die Haare bestimmten Mittel 0,05 Gew.-% bis 10 Gew.-%, insbesondere 0,5 Gew.-% bis 5 Gew.-%, bezogen auf das Gesamtgewicht des Mittels.

Erfindungsgemäße wäßrige kosmetische Reinigungsmittel oder für die wäßrige Reinigung bestimmte wasserarme oder wasserfreie Reinigungsmittelkonzentrate können anionische, nichtionische und/oder amphotere Tenside enthalten.

Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und - je nach Wunsch - für Schaumregulierung.

Bei den hydrophilen Anteilen eines Tensidmoleküls handelt es sich meist um polare funktionelle Gruppen, beispielweise -COO⁻, -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wäßriger Lösung bilden sie im sauren oder neutralen Milieu negativ geladene organische Ionen. Kationische Tenside sind beinahe ausschließlich durch das Vorhandensein einer quarternären Ammoniumgruppe gekennzeichnet. In wäßriger Lösung bilden sie im sauren oder neutralen Milieu positiv geladene organische Ionen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

| | | |
|---|---|---|
| RNH₂⁺CH₂CH₂COOH X⁻ | (bei pH=2) | X⁻ = beliebiges Anion, z.B. Cl⁻ |
| RNH₂⁺CH₂CH₂COO⁻ | (bei pH=7) | |
| RNHCH₂CH₂COO⁻ B⁺ | (bei pH=12) | B⁺ = beliebiges Kation, z.B. Na⁺ |

Typisch für nicht-ionische Tenside sind Polyether-Ketten. Nicht-ionische Tenside bilden in wäßrigem Medium keine Ionen.

### A. Anionische Tenside

Vorteilhaft zu verwendende anionische Tenside sind
Acylaminosäuren (und deren Salze), wie
1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoyl-hydrolysiertes Soja Protein und Natrium-/ Kalium-Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. Acyllactylate, Lauroyllactylat, Caproyllactylat
6. Alaninate
   Carbonsäuren und Derivate, wie

1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6-Citrat und Natrium PEG-4-Lauramidcarboxylat,
3. Ether-Carbonsäuren, beispielsweise Natriumlaureth-13-Carboxylat und Natrium PEG-6-Cocamide Carboxylat,

Phosphorsäureester und Salze, wie beispielsweise DEA-Oleth-10-Phosphat und Dilaureth-4 Phosphat,

Sulfonsäuren und Salze, wie
1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat und Dinatriumundecylenamido-MEA-Sulfosuccinat
   sowie
   Schwefelsäureester, wie

1. Alkylethersulfat, beispielsweise Natrium-, Ammonium-, Magnesium-, MIPA-, TIPA- Laurethsulfat, Natriummyrethsulfat und Natrium C₁₂₋₁₃-Parethsulfat,
2. Alkylsulfate, beispielsweise Natrium-, Ammonium- und TEA-Laurylsulfat.

### B. Kationische Tenside

Vorteilhaft zu verwendende kationische Tenside sind
1. Alkylamine,
2. Alkylimidazole,
3. Ethoxylierte Amine und
4. Quaternäre Tenside.
5. Esterquats

Quaternäre Tenside enthalten mindestens ein N-Atom, das mit 4 Alkyl- und/oder Arylgruppen kovalent verbunden ist. Dies führt, unabhängig vom pH Wert, zu einer positiven Ladung. Vorteilhafte quaternäre Tenside sind Alkylbetain, Alkylamidopropylbetain und Alkyl-amidopropylhydroxysulfain. Kationische Tenside können ferner bevorzugt im Sinne der vorliegenden Erfindung gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7. Polyglycerinester, Diglycerinester, Monoglycerinester
8. Methylglucosester, Ester von Hydroxysäuren

Vorteilhaft ist ferner die Verwendung einer Kombination von anionischen und/oder amphoteren Tensiden mit einem oder mehreren nicht-ionischen Tensiden.

Kosmetische Zubereitungen, die kosmetische Reinigungszubereitungen für die Haut darstellen, können in flüssiger oder fester Form vorliegen. Sie enthalten neben erfindungsgemäß verwendeten Wirkstoffkombinationen vorzugsweise mindestens eine anionische, nicht-ionische oder amphotere oberflächenaktive Substanz oder Gemische daraus und Hilfsmittel, wie sie üblicherweise dafür verwendet werden. Die oberflächenaktive Substanz kann in einer Konzentration zwischen 1 und 94 Gew.-% in den Reinigungszubereitungen vorliegen, bezogen auf das Gesamtgewicht der Zubereitungen.

Kosmetische Zubereitungen, die ein Shampoonierungsmittel darstellen, enthalten neben einem wirksamen Gehalt an Wirkstoffkombinationen vorzugsweise mindestens eine anionische, nicht-ionische oder amphotere oberflächenaktive Substanz oder Gemische daraus, und Hilfsmittel, wie sie üblicherweise dafür verwendet werden. Die oberflächenaktive Substanz kann in einer Konzentration zwischen 1 Gew.-% und 94 Gew.-% in dem Shampoonierungsmittel vorliegen.

Die erfindungsgemäßen Zusammensetzungen enthalten außer den vorgenannten Tensiden Wasser und gegebenenfalls die in der Kosmetik üblichen Zusatzstoffe, beispielsweise Parfüm, Verdicker, Farbstoffe, Desodorantien, antimikrobielle Stoffe, rückfettende Agentien, Komplexierungs- und Sequestrierungsagentien, Perlglanzagentien, Pflanzenextrakte, Vitamine, Wirkstoffe und dergleichen.

Es ist erfindungsgemäß bevorzugt, den erfindungsgemäß verwendeten Wirkstoffkombinationen bzw. kosmetischen oder dermatologischen Zubereitungen, solche Wirkstoffkombinationen enthaltend Komplexbildner zuzufügen.

Komplexbildner sind an sich bekannte Hilfsstoffe der Kosmetologie bzw. der medizinischen Galenik. Durch die Komplexierung von störenden Metallen wie Mn, Fe, Cu und anderer können beispielsweise unerwünschte chemische Reaktionen in kosmetischen oder dermatologischen Zubereitungen verhindert werden.

Komplexbildner, insbesondere Chelatoren, bilden mit Metallatomen Komplexe, welche bei Vorliegen eines oder mehrerer mehrbasiger Komplexbildner, also Chelatoren, Metallacyclen darstellen. Chelate stellen Verbindungen dar, in denen ein einzelner Ligand mehr als eine Koordinationsstelle an einem Zentralatom besetzt. In diesem Falle werden also normalerweise gestreckte Verbindungen durch Komplexbildung über ein Metall-Atom od. -Ion zu Ringen geschlossen. Die Zahl der gebundenen Liganden hängt von der Koordinationszahl des zentralen Metalls ab. Voraussetzung für die Chelatbildung ist, daß die mit dem Metall reagierende Verbindung zwei oder mehr Atomgruppierungen enthält, die als Elektronendonatoren wirken.

Der oder die Komplexbildner können vorteilhaft aus der Gruppe der üblichen Verbindungen gewählt werden, wobei bevorzugt mindestens eine Substanz aus der Gruppe bestehend aus Weinsäure und deren Anionen, Citronensäure und deren Anionen, Amlnopolycarbonsäuren und deren Anionen (wie beispielsweise Ethylendiamintetraessigsäure (EDTA) und deren Anionen, Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen).

Der oder die Komplexbildner sind erfindungsgemäß vorteilhaft in kosmetischen oder dermatologischen Zubereitungen bevorzugt zu 0,01 Gew.-% bis 10 Gew.-%, bevorzugt zu 0,05 Gew.-% bis 5 Gew.-%, insbesondere bevorzugt zu 0,1 - 2,0 Gew.-%, bezogen auf das Gesamtgewicht der Zubereitungen, enthalten.

Durch die kosmetischen Zubereitungen enthaltend einen wirksamen Gehalt an den erfindungsgemäß verwendeten Wirkstoffkombinationen können kosmetische oder dermatologische Zubereitungen gegen Oxidation oder Photooxidation geschüht werden, wobei diese Zubereitungen z.B. Zubereitungen zur Behandlung und Pflege der Haare darstellen, insbesondere Haarfärbemittel, Haarlacke, Shampoonierungsmittel, Farbshampoonierungsmittel, ferner Schminkprodukte wie z.B. Nagellacke, Lippenstifte, Teintgrundlagen, Wasch- und Duschzubereitungen, Cremes zur Behandlung oder Pflege der Haut oder andere kosmetische Zubereitungen, deren Bestandteile Stabilitätsprobleme aufgrund von Oxidation bzw. Photooxidation bei der Lagerung mit sich bringen können.

Vorzugsweise beträgt die Menge an erfindungsgemäß verwendeten Wirkstoffkombinationen in diesen Zubereitungen 0,01 - 30 Gew.-%, bevorzugt 0,05 - 20 Gew.-%, insbesondere 0,1 - 10,0 Gew.-%, bezogen auf das Gesamtgewicht der Zubereitungen.

Alle Mengenangaben, Anteile und Prozentanteile sind, soweit nicht anders angegeben, auf das Gewicht und die Gesamtmenge bzw. auf das Gesamtgewicht der Zubereitungen bezogen.

Die nachfolgenden Beispiele sollen die vorliegende Erfindung verdeutlichen, ohne sie einzuschränken.

### Beispiel 1

| O/W-Creme | |
|---|---|
| | Gew.-% |
| Glyceryl Stearate | 3.0000 |
| Cetyl Alcohol | 3.0000 |
| PEG-40 Stearate | 3.5000 |
| Paraffinum Liquidum | 5.0000 |
| C12-15 Alkyl Benzoate | 0.5000 |
| Cyclomethicone | 5.0000 |
| Glycerin | 5.0000 |
| Sodium Chloride | 5.0000 |
| Kreatin | 2.0000 |
| Farbstoffe/Parfüm | q.s. |
| Konservierungsmittel | |
| Wasser | ad 100.0000 |

Die Bestandteile der Ölphase werden miteinander vereinigt, dann bei 60 - 70 °C mit der ebenfalls vereinigten Wasserphase verrührt worauf das Gemisch homogenisiert wird. Hernach wird auf Raumtemperatur abgekühlt.

### Beispiel 2

| O/W Creme | |
|---|---|
| | Gew.-% |
| Glyceryl Stearate | 2.4000 |
| Cetyl Alcohol | 2.4000 |
| Glyceryl Stearate + PEG- | 1.2000 |
| 100 Stearate | |
| Paraffinum Liquidum | 15.0000 |
| Xanthan Gum | 0.2000 |
| Glycerin | 3.0000 |
| Urea | 2,0000 |
| Kreatinphosphat | 2.0000 |
| Sodium Chloride | 5.0000 |
| Diazolidinyl Urea | 0.3000 |
| Farbstoffe/Parfüm | q.s. |
| Konservierungsmittel | |
| Wasser | ad 100.0000 |

Die Bestandteile der Ölphase werden miteinander vereinigt, dann bei 60 - 70 °C mit der ebenfalls vereinigten Wasserphase verrührt worauf das Gemisch homogenisiert wird. Hernach wird auf Raumtemperatur abgekühlt.

### Beispiel 3

| O/W-Creme | |
|---|---|
| | Gew.-% |
| Cetyl Alcohol | 2.4000 |
| Steareth-21 | 1.2000 |
| Steareth-2 | 2.4000 |
| Paraffinum Liquidum | 15.0000 |
| Xanthan Gum | 0.2000 |
| Propylenglykol | 1.0000 |
| Urea | 5.0000 |
| Kreatin | 1,0000 |
| Sodium Chloride | 5.0000 |
| Diazolidinyl Urea | 0.3000 |
| Farbstoffe/Parfüm | q.s. |
| Konservierungsmittel | |
| Wasser | ad 100.0000 |

Die Bestandteile der Ölphase werden miteinander vereinigt, dann bei 60 - 70 °C mit der ebenfalls vereinigten Wasserphase verrührt worauf das Gemisch homogenisiert wird. Hernach wird auf Raumtemperatur abgekühlt.

### Beispiel 4

| O/W-Creme | |
|---|---|
| | Gew.-% |
| Cetyl Alcohol | 2.4000 |
| Steareth-21 | 1.2000 |
| Steareth-2 | 2.4000 |
| Octyldodecanol | 0.1000 |
| PPG-14 Butyl Ether | 5.0000 |
| Cyclomethicone | 5.0000 |
| Trisodium EDTA | 1.5000 |
| Butylenglykol | 2.0000 |
| Kreatin | 2.0000 |
| Natriumchlorid | 3.0000 |
| Farbstoffe/Parfüm | q.s. |
| Konservierungsmittel | |
| Wasser | ad 100.0000 |

Die Bestandteile der Ölphase werden miteinander vereinigt, dann bei 60 - 70 °C mit der ebenfalls vereinigten Wasserphase verrührt worauf das Gemisch homogenisiert wird. Hernach wird auf Raumtemperatur abgekühlt.

## Patentansprüche

1. Kosmetische oder dermatologische Zubereitungen mit einem Gehalt an
a) Kreatin, Kreatinphosphat, Kreatinsulfat, Kreatinacetat oder Kreatinascorbat
b) 0,05 - 30 Gew.-% an einem oder mehreren Elektrolyten aus der Gruppe NaCl, NaBr, Nal, Na₂B₄O₇, Na₂SiO₃, Na₂CO₃, NaHCO₃, Na₃PO₄, Na₂HPO₄, NaH₂PO₄, KCl, Kl, LiCl, NH₄Cl, ZnCl₂, Al₂SO_{4,} MgSO_{4,} Natriumliponat, Ammoniumlactat, Natriumlactat, Natriumbicarbonat, Natriumcitrat, Natriumpropionat, bezogen auf das Gesamtgewicht der Zubereitungen
c) einer oder mehreren Substanzen gewählt aus der Gruppe Harnstoff und 0.05 - 30 Gew.- % an Polyolen, gewählt aus der Gruppe Glycerin, Butylenglykole, Propylenglykole, Ethylenglykol, Pentandiole, Hexandiole, insbesondere jeweils die vicinalen Hydroxyverbindungen, Diethylenglykol, Triethylenglykol, Dipropylenglykol, Tripropylenglykol, Dibutylenglykol oder Tributylenglykol, bezogen auf das Gesamtgewicht der Zubereitungen,
mit Ausnahme folgender Zubereitungen:
| O/W Creme | |
|---|---|
| | Gew.-% |
| Paraffinöl (DAB 9) | 7,00 |
| Avocadoöl | 4,00 |
| Glycerylmonostearat | 2,00 |
| Natriumlactat | 3,00 |
| Glycerin | 3,00 |
| Konservierungsmittel, Farbstoffe, Parfüm | q.s. |
| Phosphokreatin | 0.4 |
| Wasser | ad 100,00 |
| Liposornenhaltiges Gel | |
|---|---|
| | Gew.-% |
| Lecithin | 6,00 |
| Schibutter | 3,00 |
| Taurin | 0,20 |
| Natriumcitrat | 0,50 |
| Glycin | 0,20 |
| Harnstoff | 0,20 |
| | |
|---|---|
| Natrium PCA | 0,50 |
| Hydrolysiertes Kollagen | 2,00 |
| Xanthan Gummi | 1,40 |
| Sorbitol | 3,00 |
| Konservierungsmittel, Farbstoffe, Parfüm | q.s. |
| Kreatin | 1,20 |
| Wasser | ad 100,00 |
| O/W Creme | |
|---|---|
| | Gew.-% |
| Paraffinöl (DAB 9) | 7,00 |
| Soyaöl | 4,00 |
| Glycerylmonostearat | 2,00 |
| Natriumlactat | 3,00 |
| Glycerin | 8,00 |
| Konservierungsmittel, Farbstoffe, Parfüm | q.s. |
| Phosphokreatin | 0.08 |
| Wasser | ad 100,00 |

2. Zubereitungen gemäß Anspruch 1 oder 2, **dadurch gekennzeichnet, dass** sie 1 - 5 Gew.- %, an einem oder mehreren Elektrolyten, bevorzugt Natriumchlorid enthalten, bezogen auf die Gesamtzusammensetzung der Zubereitungen.

3. Zubereitungen gemäß Anspruch 1 oder 2, **dadurch gekennzeichnet, dass** sie 0,1 Gew.-% bis 20 Gew.-%, insbesondere bevorzugt zu 1 - 15 Gew.-% an einem oder mehreren Polyolen enthalten, bezogen auf das Gesamtgewicht der Zubereitungen.

## Claims

1. Cosmetic or dermatological preparations having a content of
a) creatine, creatine phosphate, creatine sulphate, creatine acetate or creatine ascorbate,
b) 0.05 - 30% by weight of one or more electrolytes selected from the group consisting of NaCl, NaBr, Nal, Na₂B₄O₇, Na₂SiO₃, Na₂CO₃, NaHCO₃, Na₃PO₄, Na₂HPO₄, NaH₂PO₄, KCl, Kl, LiCl, NH₄Cl, ZnCl₂, Al₂SO₄, MgSO₄, sodium lipoate, ammonium lactate, sodium lactate, sodium bicarbonate, sodium citrate, sodium propionate, based on the total weight of the preparations,
c) one or more substances selected from the group consisting of urea and 0.05 - 30% by weight of polyols selected from the group consisting of glycerol, butylene glycols, propylene glycols, ethylene glycol, pentanediols, hexanediols, in particular in each case the vicinal hydroxy compounds, diethylene glycol, triethylene glycol, dipropylene glycol, tripropylene glycol, dibutylene glycol and tributylene glycol, based on the total weight of the preparations,
with the exception of the following preparations:
| O/W cream | |
|---|---|
| | % by wt. |
| Paraffin oil (DAB 9) | 7.00 |
| Avocado oil | 4.00 |
| Glyceryl monostearate | 2.00 |
| Sodium lactate | 3.00 |
| Glycerol | 3.00 |
| Preservatives, dyes, perfume | q.s. |
| Phosphocreatine | 0.4 |
| Water | ad 100.00 |
| Liposome-containing gel | |
|---|---|
| | % by wt. |
| Lecithin | 6.00 |
| Shea butter | 3.00 |
| Taurine | 0.20 |
| Sodium citrate | 0.50 |
| Glycine | 0.20 |
| Urea | 0.20 |
| Sodium PCA | 0.50 |
| Hydrolysed collagen | 2.00 |
| Xanthan gum | 1.40 |
| Sorbitol | 3.00 |
| Preservatives, dyes, perfume | q.s. |
| Creatine | 1.20 |
| Water | ad 100.00 |
| O/W cream | |
|---|---|
| | % by wt. |
| Paraffin oil (DAB 9) | 7.00 |
| Soybean oil | 4.00 |
| Glyceryl monostearate | 2.00 |
| Sodium lactate | 3.00 |
| Glycerol | 8.00 |
| Preservatives, dyes, perfume | q.s. |
| Phosphocreatine | 0.08 |
| Water | ad 100.00 |

2. Preparations according to Claim 1 or 2,
**characterized in that** they contain 1 - 5% by weight of one or more electrolytes, preferably sodium chloride, based on the total composition of the preparations.

3. Preparations according to Claim 1 or 2,
**characterized in that** they contain 0.1% by weight to 20% by weight, particularly preferably 1 - 15% by weight of one or more polyols, based on the total weight of the preparations.

## Revendications

1. Préparations cosmétiques ou dermatologiques présentant une teneur en
a) créatine, phosphate de créatine, sulfate de créatine, acétate de créatine ou ascorbate de créatine,
b) de 0,05 à 30 % en poids d'un ou de plusieurs électrolytes parmi le groupe constitué du NaCl, du NaBr, du Nal, du Na₂B₄O₇, du Na₂SiO₃, du Na₂CO₃, du NaHCO₃, du Na₃PO₄, du Na₂HPO₄, du NaH₂PO₄, du KCl, du Kl, du LiCl, du NH₄Cl, du ZnCl₂, de l'Al₂SO₄, du MgSO₄, du lipoate de sodium, du lactate d'ammonium, du lactate de sodium, du bicarbonate de sodium, du citrate de sodium, du propionate de sodium, par rapport au poids total des préparations,
c) une ou plusieurs substances choisies parmi le groupe constitué de l'urée et de 0,05 à 30 % en poids de polyols choisis parmi le groupe constitué du glycérol, de butylèneglycols, de propylèneglycols, de l'éthylèneglycol, de pentanediols, d'hexanediols, en particulier dans chaque cas des composés hydroxy voisins, du diéthylèneglycol, du triéthylèneglycol, du dipropylèneglycol, du tripropylèneglycol, du dibutylèneglycol ou du tributylèneglycol, dans chaque cas par rapport au poids total des préparations,
à l'exception des préparations suivantes :
| Crème H/E | |
|---|---|
| | % en poids |
| Huile de paraffine (DAB 9) | 7,00 |
| Huile d'avocat | 4,00 |
| Monostéarate de glycéryle | 2,00 |
| Lactate de sodium | 3,00 |
| Glycérol | 3,00 |
| Conservateurs, colorants, parfum | q.s. |
| Phosphocréatine | 0,4 |
| Eau | ad 100,00 |
| Gel contenant des liposomes | |
|---|---|
| | % en poids |
| Lécithine | 6,00 |
| Beurre de karité | 3,00 |
| Taurine | 0,20 |
| Citrate de sodium | 0,50 |
| Glycine | 0,20 |
| Urée | 0,20 |
| PCA sodique | 0,50 |
| Collagène hydrolysé | 2,00 |
| Gomme de xanthane | 1,40 |
| Sorbitol | 3,00 |
| Conservateurs, colorants, parfum | q.s. |
| Créatine | 1,20 |
| Eau | ad 100,00 |
| Crème H/E | |
|---|---|
| | % en poids |
| Huile de paraffine (DAB 9) | 7,00 |
| Huile de soja | 4,00 |
| Monostéarate de glycéryle | 2,00 |
| Lactate de sodium | 3,00 |
| Glycérol | 8,00 |
| Conservateurs, colorants, parfum | q.s. |
| Phosphocréatine | 0,08 |
| Eau | ad 100,00 |

2. Préparations selon la revendication 1 ou 2, **caractérisées en ce qu'**elles comprennent de 1 à 5 % en poids d'un ou de plusieurs électrolytes, de préférence de chlorure de sodium, par rapport à la composition totale des préparations.

3. Préparations selon la revendication 1 ou 2, **caractérisées en ce qu'**elles comprennent de 0,1 % en poids à 20 % en poids, en particulier de 1 à 15 % en poids d'un ou de plusieurs polyols, dans chaque cas par rapport au poids total des préparations.
